## Europäisches Patentamt
## European Patent Office
## Office européen des brevets

(11) Veröffentlichungsnummer: **0 020 290**
**B1**

(12) **EUROPÄISCHE PATENTSCHRIFT**

(45) Veröffentlichungstag der Patentschrift:
**11.07.84**

(21) Anmeldenummer: **80730038.9**

(22) Anmeldetag: **30.05.80**

(51) Int. Cl.³: **C 12 P  21/06**, C 12 N  9/58 //
C07C103/52

(54) Verfahren zur spezifischen Abspaltung von Proteinsequenzen aus Proteinen.

(30) Priorität: **31.05.79  DE 2922496**
**27.03.80  DE 3012170**
**27.03.80  DE 3012169**

(43) Veröffentlichungstag der Anmeldung:
**10.12.80 Patentblatt 80/25**

(45) Bekanntmachung des Hinweises auf die Patenterteilung:
**11.07.84 Patentblatt 84/28**

(84) Benannte Vertragsstaaten:
**AT BE CH DE FR GB IT LI LU NL SE**

(73) Patentinhaber: **SCHERING AKTIENGESELLSCHAFT**
**Berlin und Bergkamen,**
**Müllerstrasse 170/178 Postfach 65 03 11,**
**D-1000 Berlin 65 (DE)**

(72) Erfinder: **Daum, Joachim, Dr., Kyllmannstrasse 22 d,**
**D-1000 Berlin 45 (DE)**
Erfinder: **Siewert, Gerhard, Dr., Im Fischgrund 50 c,**
**D-1000 Berlin 28 (DE)**
Erfinder: **Töpert, Michael, Dr., Askaloner Weg 11,**
**D-1000 Berlin 28 (DE)**
Erfinder: **Seliger, Hartmut, Prof. Dr., Öschwende 55,**
**D-7900 Ulm-Lehr (DE)**

(56) Entgegenhaltungen:
EP - A - 0 001 930
DE - A - 1 442 147

CHEMICAL ABSTRACTS, Band 77, Nr. 9, 28. August 1972, Seite 190, Nr. 58236n Columbus, Ohio, U.S.A. M.E. SOBERNO et al.: "Specificity of bacterial collagenase. Studies with peptides newly synthesized using the solid-phase method"
CHEMICAL ABSTRACTS, Band 70, Nr. 13, 31. März 1969, Seite 29, Nr. 54163u Columbus, Ohio, U.S.A. J.T. POTTS et al.: "Limited proteolysis by exopeptidases"
CHEMICAL ABSTRACTS, Band 89, Nr. 13, 25. September 1978, Seite 316, Nr. 102526f Columbus, Ohio, U.S.A. T. YOSHIMOTO et al.: "Post-Proline cleaving enzyme and

(56) Entgegenhaltungen: (Fortsetzung)
post-proline dipeptidyl aminopeptidase. Comparison of two peptidases with high specificity for proline residues"
CHEMICAL ABSTRACTS, Band 69, Nr. 21, 18. November 1968, Seite 780, Spalte 1, Nr. 83517x Columbus, Ohio, U.S.A. A. YARON et al.: "Aminopeptidase-P"

## Beschreibung

Die vorliegende Erfindung besteht in einem Verfahren zur Herstellung von Proteinen, das dadurch gekennzeichnet ist, dass man von einem Protein, das die Tetrapeptidsequenz Pro-Xyz-Gly-Pro enthält, wobei Xyz jede beliebige Aminosäure bedeuten kann, enzymatisch die C-terminal auf die Tetrapeptidsequenz folgende Proteinsequenz abspaltet.

Bei der Synthese von Fremdproteinen (z.B. Säugetier-Proteohormone) durch genetisch modifizierte Mikroorganismen wird das Fremdgen, das die gewünschte Proteinsequenz codiert und das am 3-Ende des codogenen Stranges ein oder mehrere Stopcodons enthält, in ein Strukturgen des Mikroorganismus eingebaut. Dadurch bleiben die Regulationsorte auf der mikrobiellen DNA funktionsfähig und die Proteinbiosynthese kann in üblicher Weise ablaufen. Als Primärprodukt der Proteinsynthese wird dann ein Fusionsprotein erhalten, das aminoterminal eine mehr oder weniger lange Sequenz des mikrobiellen Proteins und carboxylterminal das gewünschte Fremdprotein enthält. Bei der sogenannten «direkten Synthese», bei der das Fremdgen direkt an das mikrobielle Startcodon für Methionin angekuppelt wird, entsteht ein Fusionsprotein, das gegenüber dem Fremdprotein aminoterminal um Methionin verlängert ist. Das Fremdprotein muss erst aus diesem Fusionsprotein durch eine proteinchemische Reaktion abgespalten werden. Die einzige für diese Spaltung zur Zeit bekannte Methode ist die Reaktion mit Bromcyan, die zu einer Spaltung der Peptidsequenz am Carboxylende von Methionin-Resten führt (S.B. Needleman, protein sequence determination, Springer Verlag, 1970, N.Y.). Dieses Verfahren versagt jedoch, wenn in dem gewünschten Fremdprotein weitere Methionin-Reste vorkommen. Zusätzlich hat die Bromcyanspaltung den Nachteil, an verschiedenen anderen Aminosäuren zu Nebenreaktionen zu führen.

Es wurde nun gefunden, dass eine Gewinnung des Fremdproteins ohne übliche Nebenreaktionen auch in Gegenwart von Methionin-Resten durch eine selektive enzymatische Hydrolyse gelingt, wenn in dem Fusionsprotein am aminoterminalen Ende der Fremdprotein-Sequenz ein Tetrapeptid der allgemeinen Formel Pro-Xyz-Gly-Pro, wobei Xyz jede beliebige Aminosäure sein kann, steht. Zunächst wird selektiv die Xyz-Gly-Bindung mit einer Collagenase (E.C. 3.4.24.3.) gespalten und anschliessend der Glycin-Rest mit einer Aminoacylprolinaminopeptidase (Aminopeptidase-P, E.C. 3.4.11.9.) und der Prolin-Rest mit einer Prolinaminopeptidase (E.C. 3.4.11.5.) entfernt.

Das erfindungsgemässe Verfahren besteht daher aus der spezifischen enzymatischen Spaltung eines, durch Genetic Engineering erhaltenen Fusionsprotein, das an der Verknüpfungsstelle von originaler Proteinsequenz und Fremdprotein eine Collagenase-spezifische Tetrapeptidsequenz der allgemeinen Formel Pro-Xyz-Gly-Pro hat, wobei für die Enzymatische Spaltung die Enzyme Collagenase, Aminoacylprolinaminopeptidase und Prolinaminopeptidase verwendet werden.

Unter Xyz versteht man alle natürlich vorkommenden Aminosäuren, die im genetischen Code enthalten sind.

Die für die enzymatische Abspaltung des gewünschten Proteins aus dem Fusionsprotein erforderliche Collagenase muss weitgehend frei von anderen Proteasen sein. Das Enzym wird z.B. von Clostridium histolyticum produziert und kann durch Fermentation in üblicher Weise nach bekannten Verfahren gewonnen werden. Es ist in gereinigter Form auch kommerziell erhältlich. Zur Blockierung der in den meisten Präparationen noch in geringer Menge enthaltenen Proteasen wird bei der Inkubation mit dem Fusionsprotein ein Proteaseinhibitor (z.B. Diisopropylfluorphosphat, N-Äthylmaleinimid oder Phenylmethylsulfonylfluorid) zugesetzt. Collagenase selbst wird durch diesen Inhibitor nicht gehemmt. Bei dieser Umsetzung entsteht das um die Sequenz Gly-Pro aminoterminal verlängerte gewünschte Fremdprotein. Es kann aus dem Reaktionsgemisch abgetrennt werden. Die dafür einzusetzenden Methoden richten sich nach den speziellen Eigenschaften der Verbindung, die im wesentlichen von dem gewünschten Fremdprotein (z.B. Proinsulin, Insulin A- oder B-Kette, ACTH, STH) bestimmt werden.

Die Abspaltung des Glycin- und des Prolin-Restes mit den beiden spezifischen Aminopeptidasen kann in zwei getrennten Hydrolyseansätzen nacheinander oder wie in den meisten Fällen auch durch gemeinsame Inkubation mit beiden Enzymen erreicht werden. Die Aminopeptidase-P kann aus einem Escherichia coli-Stamm, z.B. dem Stamm B wie in Biochem. and Biophys. Res. Comm. Vol. 32, 658–663 (1968) beschrieben durch Ammonsulfat-Fraktionierung, Aceton-Fällung sowie durch Chromatographie an Calciumphosphat, DEAE-Cellulose und Sephadex G–200 isoliert werden. Die Prolinaminopeptidase wird ebenfalls aus einem Escherichia coli-Stamm, z.B. aus dem Stamm K 12 isoliert. Sie wird nach Ammonsulfat-Fraktionierung und zweimaliger Chromatographie an DEAE-Cellulose ausreichend rein erhalten. Sie muss frei von weniger spezifischen Aminopeptidase sein [J. Biol. Chem. 234, 1740–1746 (1959); ibid. 237, 2207–2212 (1962)].

Die enzymatischen Spaltungen werden abhängig von den Lösungseigenschaften des eingesetzten Fusionsproteins in einem geeigneten Puffer, wie z.B. Tris- oder Phosphat-Puffer, bei pH 6–9 durchgeführt. Die spezifischen Enzyme werden hinzugegeben und die sich anschliessende Inkubation wird am besten bei 25–40 °C in 0,5–10 Stunden durchgeführt.

Es wurde ausserdem gefunden, dass die Dipeptidsequenz Gly-Pro, die sich nach der Spaltung der Xyz-Gly-Bindung der N-terminalen Tetrapeptidsequenz des Fusionproteins mit einer Collagenase noch am aminoterminalen Ende der Fremdproteinsequenz befindet, in einem Schritt mit einer Postprolindipeptidylaminopeptidase (E.C. 3.4.14.1.) abgespalten werden kann. Diese Postprolindipeptidylaminopeptidase ist entsprechend den Angaben in der Literatur leicht zugänglich [Biochim. Biophys. Acta 485, 391 (1977)].

Die Vorteile bei der Verwendung dieses Enzyms liegen in der höheren Aktivität dieser Dipeptidylaminopeptidase und in der bequemen Aufarbeitung des Endproduktes. Die hohe Aktivität des Enzyms erlaubt einen sehr rationellen Einsatz des Verfahrens. Sie bleibt auch bei einer Trägerbindung des Enzyms erhalten, wodurch sich das Verfahren wesentlich vereinfacht. Da die Abspaltung der beiden Aminosäuren in einem Schritt erfolgt, ist das Endprodukt bei einer unvollständigen Spaltung nicht von zwei Nebenprodukten (Ausgangsprodukt und dem um eine Aminosäure verkürzten Ausgangsprodukt), sondern nur von einem Ausgangsprodukt bei der Endreinigung abzutrennen. Dieses Verfahren ist jedoch nicht einsetzbar, wenn die gewünschte Fremdprotein-Sequenz mit Prolin oder mit Aminoacyl-prolin beginnt.

Mit diesem Verfahren lässt sich nicht nur Gly-Pro, sondern lassen sich auch alle aminoterminal gebundenen Dipeptidsequenzen Yzx-Pro von einem Protein abspalten, da Postprolindipeptidylaminopeptidase nicht spezifisch hinsichtlich der vor dem Prolin stehenden Aminosäure ist. Das Verfahren kann auch bei dem Prinzip der sogenannten «direkten Synthese» zur Überführung des primären Fusionsproteins in das gewünschte Fremdprotein eingesetzt werden. In das Fremdgen muss dann zwischen dem als Starter notwendigen Codon für Methionin und der das Protein codierenden Nucleotidsequenz ein Codon für Prolin eingebaut werden. Als Primärprodukt wird dann das aminoterminal um Met-Pro verlängerte Protein erhalten, von dem die Met-Pro-Sequenz leicht mit der Postprolindipeptidylaminopeptidase abgespalten werden kann. Auch hier kann man das Verfahren nur einsetzen, wenn die auf das Prolin C-terminal folgende Peptidsequenz nicht mit Prolin oder Aminoacylprolin beginnt.

Unter Yzx versteht man alle natürlich vorkommenden Aminosäuren, die im genetischen Code enthalten sind. Im folgenden versteht man unter Vwu alle natürlich vorkommenden Aminosäuren bis auf Prolin sowie alle Dipeptide bis auf Aminoacylprolin.

Die Abspaltung der aminoterminalen Reste Gly-Pro, Met-Pro bzw. Yzx-Pro, wobei Yzx die obenangegebene Bedeutung hat, von den Fusionsproteinen mit der N-terminalen Peptidsequenz Yzx-Pro-Vwu mit Postprolindipeptidylaminopeptidase geht folgendermassen vonstatten: Das Fusionsprotein wird in einem geeigneten Puffer, wie z.B. Tris-Puffer oder Phosphatpuffer (wobei der pH-Wert besonders günstig im Bereich von pH7–pH8 ist), abhängig von den Lösungseigenschaften des Fusionsproteins, gelöst und bei 30–40°, vorzugsweise um 37 °C mit der PPDA inkubiert. Die Reaktionszeit wird weitgehend durch das Fusionsprotein bestimmt, in der Mehrzahl der Fälle genügen 3–5 Stunden.

Wenn das gewünschte Protein einen N-terminalen Prolin-Rest haben soll, kann das erfindungsgemässe Verfahren in analoger Weise angewendet werden. Im Fall des Fusionsproteins stellt dieser N-terminale Prolin-Rest bereits den carboxylterminal auf Gly folgenden Prolin-Rest dar. Aus der Collagenase-spezifischen Sequenz Pro-Xyz-Gly-Pro werden mit Collagenase und Aminoacylprolin-Aminopeptidase Pro-Xyz und Gly abgespalten.

In der vorliegenden Erfindung wird durch die Einführung einer weiteren Aminosäure Zxy (mit Ausnahme von Prolin) zwischen der Tetrapeptidsequenz Pro-Xyz-Gly-Pro, wobei Xyz jede beliebige Aminosäure darstellen kann, und dem gewünschten Fremdprotein auch die Herstellung von Fremdproteinen mit Prolin als zweiter N-terminaler Aminosäure aus Fusionsproteinen nach primärer Collagenase-Spaltung der Xyz-Gly-Bindung mit der vorteilhaften Postprolindipeptidylaminopeptidase-Spaltung der Pro-Zxy-Bindung und anschliessender Abtrennung der Aminosäure Zxy mit Leucinaminopeptidase [LAP, E.C. 3.4.11.1., α-Aminoacylpeptidhydrolase] ermöglicht.

Enthalten z.B. die gewünschten Fremdproteine N-terminal als zweite Aminosäure noch ein weiteres Prolin, wie das beim humanen Wachstumshormon oder beim Rinder-prolactin der Fall ist, so würde PPDA die Verdauung bis zu einem Protein fortsetzen, das um das Prolin-haltige Dipeptid kürzer wäre.

Es wurde nun gefunden, dass mit dem vorgenannten Verfahren auch Fremdproteine mit Prolin als zweiter N-terminaler Aminosäure aus Fusionsproteinen gewonnen werden können, wenn zwischen der Tetrapeptidsequenz Pro-Xyz-Gly-Pro, wobei Xyz jede beliebige Aminosäure darstellen kann, und dem gewünschten Fremdprotein eine weitere Aminosäure Zxy, die jede beliebige Aminosäure mit Ausnahme von Prolin bedeuten kann, eingefügt wird. Nach der Collagenase-Spaltung der Xyz-Gly-Bindung der Pentapeptidsequenz Pro-Xyz-Gly-Pro-Zxy kann man nun mit dem Enzym PPDA das Dipeptid Gly-Pro abspalten, ohne dass das Prolin-haltige Fremdprotein angegriffen wird. Danach erfolgt die Abspaltung der «Schutzgruppe» Zxy auf enzymatischem Wege mit LAP.

Das N-terminal um eine Aminosäure verlängerte Fremdprotein wird in einem geeigneten Puffer wie z.B. Tris-Puffer oder Phosphat-Puffer (pH8 –10, vorzugsweise 8,6) zur Aktivierung der LAP, üblicherweise mit 1–5 mMol/l : MnCl₂ versetzt, gelöst und nach Zugabe der LAP bei 35–40°, vorzugsweise bei 40°, inkubiert. Die Reaktionszeit hängt entscheidend von der abzuspaltenden N-terminalen Aminosäure ab und kann zwischen wenigen Minuten und einigen Stunden liegen.

Durch die Einführung einer weiteren Aminosäure Zxy, mit der obenangegebenen Bedeutung, wie z.B. Leucin oder Glycin, zwischen die Collagenase-spezifische Sequenz und die Fremdproteinsequenz können die Vorteile der hohen Enzymaktivität von PPDA und der leichteren Reinigung der Spaltprodukte auf die Gewinnung von gewünschten Fremdproteinen, mit der N-terminalen Sequenz Uvw-Pro (mit Uvw in der Bedeutung jeder beliebigen Aminosäure mit Ausnahme von Prolin) ausgedehnt werden.

In diesem Fall muss zwischen die Collagenase-spezifische Nucleinsäure-Sequenz und die das Fremdprotein codierende Nucleinsäure-Sequenz noch ein zusätzliches Codon für die Aminosäure Zxy eingeführt werden.

Unter den Aminosäuren Zxy versteht man alle natürlich vorkommenden Aminosäuren ausser Prolin, die im genetischen Code enthalten sind.

Das in freier Form vorliegende gewünschte Fremdprotein wird nach den üblichen Methoden der Proteinreinigung in reiner Form isoliert.

Die dabei eingesetzten Verfahren werden von den Eigenschaften des Fremdproteins (z. B. Proinsulin, Insulin A- oder B-Kette, ACTH, STH) und den Eigenschaften der abzutrennenden Verbindungen bestimmt.

Aus den Beispielen ist ersichtlich, dass das Enzym Postprolindipeptidylaminopeptidase nicht nur Modellpeptide, sondern auch höher molekulare Proteine als Substrat akzeptiert.

Beispiel 1

140 mg Z-Gly-Pro-Leu-Gly-Pro-Insulin-A-Kette (50 µmol) werden in 10 ml 0,5 mol/l Tris-Puffer (pH 7,2) gelöst, der 0,1 mol/l Calcium-Acetat und zur Verhinderung einer Hydrolyse an unerwünschter Stelle durch Verunreinigung der Collagenase durch andere Proteasen 0,1 mmol/l Diisopropyl-fluorphosphat enthält, und mit 10 mg Collagenase versetzt. Nach 60 min bei 28 °C wird die Reaktion durch Zugabe von Äthanol gestoppt. Nach dem Abfiltrieren von ausgefälltem Enzym wird der Alkohol im Vakuum weitgehend abgedampft und die Lösung durch Chromatographie an Sephadex G–15 mit 0,01 mol/l Ammoniumhydrogencarbonat als Elutionsmittel entsalzt. Das Eluat wird im Vakuum auf ein kleines Volumen eingeengt und gefriergetrocknet. Ausbeute 110 mg Gly-Pro-Insulin-A-Kette. Mittels Endgruppenbestimmung (DNP-Methode) kann Glycin als N-terminale Aminosäure nachgewiesen werden. Das erhaltene Produkt wird direkt zur weiteren Umsetzung eingesetzt.

Beispiel 2

Getrennte Abspaltung der ersten beiden N-terminalen Aminosäuren 50 mg Gly-Pro-Insulin-A-Kette werden in 1 ml 0,1 mol/l Tris-Puffer (pH 8,5), der 0,5 mmol/l $MnCl_2$ enthält, gelöst. 2 µg Aminopeptidase-P werden zugegeben und 1 h bei 37 °C inkubiert. Durch kurzes Erhitzen auf 60 °C wird inaktiviert und ohne weitere Reinigung 20 µg Prolin-aminopeptidase zugegeben. Nach 24 h bei 37 °C werden die hochmolekularen Proteine durch Ausfällen mit Alkohol und Filtrieren entfernt. Das Filtrat wird im Vakuum eingeengt, in 0,01 mol/l Ammoniumhydrogencarbonat-Puffer (pH 9), aufgenommen und durch Chromatographie an Sephadex G–25 entsalzt. Die Insulin-A-Ketten-Fraktion wird direkt auf eine AE-Cellulose-Säule aufgebracht und mit einem Ammoniumhydrogencarbonat-Gradienten (0,01–0,1 mol/l, pH9) chromatographiert. Durch Einengen und Gefriertrocknen der Insulin-A-Ketten-Fraktion werden 40 mg Indulin-A-Kette erhalten.

Aminosäureanalyse:
berechnet:
$Asp_{2.00}Thr_{1.00}Ser_{2.00}Glu_{4.00}Gly_{1.00}$
$Cys_{4.00}Val_{1.00}Ile_{2.00}Leu_{2.00}Tyr_{2.00}$
gefunden:
$Asp_{1.98}Thr_{0.92}Ser_{1.64}Glu_{3.79}Gly_{1.02}$
$Cysn.b.Val_{0.98}Ile_{1.99}Leu_{2.04}Tyr_{1.80}$

Beispiel 3

Gemeinsame Abspaltung der ersten beiden N-terminalen-Aminosäuren. Wie in Beispiel 2 werden 50 mg Gly-Pro-Insulin-A-Kette in 1 ml Tris-Puffer gelöst. Anschliessend werden 2 µg Aminopeptidase-P und 20 µg Prolinaminopeptidase zugesetzt. Nach einer Reaktionszeit von 18 h bei 37 °C wird mit Alkohol gefällt und wie im Beispiel 2 aufgearbeitet.

Ausbeute 30 mg Insulin-A-Kette.

Beispiel 4

200 mg Z-Gly-Pro-Leu-Gly-Pro-Insulin-B-Kette werden wie in Beispiel 1 umgesetzt.

Ausbeute 150 mg an Gly-Pro-Insulin-B-Kette, die ohne weitere Reinigung weiter verarbeitet werden.

Beispiel 5

100 mg nach Beispiel 4 hergestellte Gly-Pro-Insulin-B-Kette werden in 5 ml Tris-Puffer gelöst und wie in Beispiel 2 nacheinander mit Aminopeptidase-P und Prolinaminopeptidase inkubiert, an Sephadex G–25 entsalzt und durch Chromatographie gereinigt.

Ausbeute 70 mg Insulin-B-Kette.

Aminosäurenanalyse:
berechnet:
$Asp_{1.00}Thr_{1.00}Ser_{1.00}Glu_{3.00}Gly_{3.00}$
$Pro_{1.00}Cys_{2.00}Phe_{3.00}Val_{3.00}Leu_{4.00}$
$Ala_{2.00}Tyr_{2.00}His_{2.00}Lys_{1.00}Arg_{1.00}$
gefunden:
$Asp_{0.95}Thr_{0.92}Ser_{0.83}Glu_{3.12}Gly_{3.02}$
$Pro_{0.98}Cys_{1.75}Phe_{2.98}Val_{3.10}Leu_{3.96}$
$Ala_{2.04}Tyr_{1.89}His_{1.94}Lys_{1.06}Arg_{0.97}$

Beispiel 6

15 mg Z-Gly-Pro-Gly-Gly-Pro-Ala-Met-Glu-His-Phe-Arg-Trp-Gly werden wie in Beispiel 1 umgesetzt.

Ausbeute 10 mg Gly-Pro-Ala-Met-Glu-His-Phe-Arg-Trp-Gly.

Beispiel 7

10 mg nach Beispiel 6 hergestelltes Gly-Pro-Ala-Met-Glu-His-Phe-Arg-Trp-Gly werden wie in Beispiel 3 mit Aminopeptidase-P und Prolinaminopeptidase gemeinsam umgesetzt. Zur Reinigung wird nach dem Ausfällen der Enzyme mit Äthanol auf ein kleines Volumen eingeengt und über Carboxymethylcellulose mit einem Ammoniumacetat-Gradienten (0,01 bis 0,2 mol/l) chromatographiert.

Ausbeute 7 mg Ala-Met-Glu-His-Phe-Arg-Trp-Gly.

Aminosäureanalyse:
berechnet:
$Glu_{1.00}Gly_{1.00}Phe_{1.00}Ala_{1.00}$
$Met_{1.00}His_{1.00}Arg_{1.00}Trp_{1.00}$
gefunden:
$Glu_{1.03}Gly_{1.01}Phe_{0.98}Ala_{1.02}$
$Met_{0.81}His_{0.93}Arg_{0.97}Trpn.b.$

## Beispiel 8

50 mg Gly-Pro-Ala werden mit 700 µg Postprolindipeptidylaminopeptidase (im folgenden als PPDA bezeichnet) in 2 ml Tris-Puffer (20 mMol/l, pH 7,8) 3 h bei 37 °C inkubiert. Der Spaltansatz wird zur Entfernung des Enzyms mit 1 ml Äthanol versetzt und das ausgefällte Enzym abfiltriert. Das Filtrat wird nach dem Einengen in $NH_4HCO_3$-Puffer (0,01 Mol/l, pH 7,4) aufgenommen und zur Trennung des Gemisches über eine Cellulose-Säule chromatographiert. Nach Zusammenfassen der entsprechenden Fraktionen wird im Vakuum eingeengt und 2mal gefriergetrocknet.
Ausbeute 13 mg Alanin (70%).

## Beispiel 9

75 mg Gly-Pro-Ser-Tyr-βNA werden wie im Beispiel 8 mit 700 µg PPDA inkubiert. Zur Trennung des Gemisches wird das gebildete Ser-Tyr-βNA mit Essigester extrahiert und nach dem Einengen 2mal aus Methanol/Äther umgefällt.
Ausbeute 43 mg (74%) Ser-Tyr-βNA Fp. $\sim$205 (Zersetzung).

## Beispiel 10

50 mg Gly-Pro-Insulin-A-Ketten-tetra-S-sulfonat (Rind) werden in 2 ml Tris-Puffer (0,1 Mol/l, pH 8,0) gelöst und mit 100 µg PPDA versetzt. Nach 4 h bei 37 °C wird über Sephadex G50 mit 0,01 Mol/l $NH_4HCO_3$ (pH 7,5) als Elutionsmittel chromatographiert. Nach Zusammenfassen der entsprechenden Fraktionen wird auf ein kleines Volumen eingeengt und 2mal gefriergetrocknet.
Ausbeute 43 mg Insulin-A-Kette.

Das Produkt ist im Gegensatz zum Ausgangsprodukt mit Leucinaminopeptidase (LAP) abbaubar.

Aminosäureanalyse:
berechnet:
$Asp_{2.00}Ser_{2.00}Gln_{4.00}Gly_{1.00}Cys_{4.00}Val_{2.00}$
$Ala_{1.00}Ile_{1.00}Leu_{2.00}Tyr_{2.00}Pro_{0.00}$
gefunden:
$Asp_{1.97}Ser_{1.70}Gln_{4.02}Gly_{1.05}Cysn.b.Val_{1.99}$
$Ala_{0.97}Ile_{1.04}Leu_{2.08}Tyr_{1.78}Pro_{0.04}$

## Beispiel 11

20 mg Prolactin-hexa-S-sulfonat werden in 5 ml Tris-Puffer (0,5 Mol/l, pH 7,2) gelöst und mit 0,1 Mol/l Calciumacetat und 0,1 mMol/l Diisopropylfluorphosphat sowie mit 5 mg Collagenase versetzt. Nach 30 Min. bei 30 °C wird die Reaktion durch Zugabe von Äthanol gestoppt. Nach Einengen im Vakuum wird in 0,02 Mol/l Tris-Puffer, pH 7,8, aufgenommen und über eine Sephadex-Säule G75 mit dem gleichen Puffer als Elutionsmittel chromatographiert. Die zusammengefassten Fraktionen werden durch Dialyse gegen entsalztes Wasser entsalzt und nach Gefriertrocknung in 4 ml Tris-Puffer (0,1 Mol/l, pH 7,9) aufgenommen und mit 5 µg PPDA 2 h bei 37 °C inkubiert. Es wird erneut über Sephadex G75 chromatographiert, die entsprechenden Fraktionen zusammengefasst, dialysiert und gefriergetrocknet.
Ausbeute 10 mg Des-Octapeptid-Prolactin-penta-S-sulfonat.

Im Gegensatz zum Ausgangsprodukt lässt sich Gly als N-terminale Aminosäure nachweisen. Weiterhin ist das Endprodukt im Gegensatz zum Zwischenprodukt mit LAP abbaubar.

## Beispiel 12

150 mg Met-Pro-Ser-Tyr-βNA werden mit 1 mg PPDA wie in Beispiel 8 umgesetzt und aufgearbeitet.
Ausbeute 80 mg (70%) Ser-Tyr-βNA Fp. $\sim$208 (Zersetzung).

## Beispiel 13

500 mg Met-Gly-Pro-amid werden mit 1 mg aktivierter LAP in 20 ml Tris-Puffer (30 mMol/l Tris·HCl, 2 mMol/l Mn $Cl_2$, pH 8,6) 30 Min. bei 40 °C inkubiert. Gly-Pro-amid wird in der Kälte mit Essigester ausgeschüttelt und nach dem Einengen im Vakuum 2mal aus Äthanol/Petroäther umgefällt.
Ausbeute 242 mg $\triangleq$ 72%, Fp. 209–210 °C (Z), $[\alpha]_D = 194,4$ (c = 1, $H_2O$).

## Beispiel 14

100 mg Rinder-Wachstumshormon werden mit 25 µg aktivierter LAP in 10 ml Tris-Puffer (30 mMol/l Tris · HCl, 2 mMol/l Mn $Cl_2$, pH 8,6) für 30 Min. bei 40 °C inkubiert. Anschliessend wird das Protein mit Äthanol gefällt, in 1 ml eines Bicarbonatpuffers (10 mMol/l $NH_4HCO_3$, pH 7,5), aufgenommen und das Gemisch bei 4 °C über eine Sephadex-G50-Säure (100 cm × 1 cm $\varnothing$) mit dem gleichen Bicarbonatpuffer getrennt. Die das Des-Alanyl[1]-Wachstumshormon enthaltenden Fraktionen werden zusammengefasst, eingeengt und gefriergetrocknet. Nach Dansylierung und saurer Hydrolyse wird dünnschichtchromatographisch als endständige Aminosäure Phenylalanin ermittelt.
Ausbeute 84 mg $\triangleq$ 84%.

## Beispiel 15

100 mg Gly-Pro-Leu-Gly-Pro-amid werden mit 1 mg PPDA in 5 ml Tris-Puffer (20 mMol/l Tris · HCL, pH 7,3) für 3 h bei 37 °C inkubiert. Das entstandene Leu-Gly-Pro-amid wird in der Kälte mit Essigester ausgeschüttelt. Nach dem Einengen wird in Tris-Puffer (30 mMol/l Tris HCl, 2 mMol/l Mn $Cl_2$, pH 8,6) aufgenommen mit 0,1 mg aktivierter LAP versetzt und für 30 Min. bei 40 °C inkubiert. Das Endprodukt wird in der Kälte mit Essigester ausgeschüttelt und nach dem Einengen im Vakuum 2mal aus Äthanol/Petroäther gefällt.

Ausbeute 18,3 mg ≙ 47%, Fp. 209–210 °C (Z), $[\alpha]_D$ = 194,4 (c = 1, $H_2O$).

## Patentansprüche

1. Verfahren zur Herstellung von Proteinen, dadurch gekennzeichnet, dass man von einem Fusionsprotein, das die N-terminale Tetrapeptidsequenz Pro-Xyz-Gly-Pro enthält, wobei Xyz jede beliebige Aminosäure bedeuten kann, enzymatisch die C-terminal auf die Tetrapeptidsequenz folgende Proteinsequenz abspaltet.

2. Verfahren zur Herstellung von Proteinen nach Anspruch 1, dadurch gekennzeichnet, dass man die Xyz-Gly-Bindung in der Tetrapeptidsequenz mit einer Collagenase selektiv spaltet und anschliessend den Glycin-Rest mit einer Aminoacylprolin-aminopeptidase und den Prolin-Rest mit einer Prolinaminopeptidase entfernt.

3. Verfahren zur Herstellung von Proteinen nach Anspruch 2, dadurch gekennzeichnet, dass man die Abspaltung des Glycin-Restes mit einer Aminoacylprolin-aminopeptidase und des Prolin-Restes mit Prolin-aminopeptidase in einem Schritt vornimmt.

4. Verfahren zur Herstellung von Proteinen nach Anspruch 2, dadurch gekennzeichnet, dass man den Gly-Pro-Rest mit einer Postprolindipeptidylaminopeptidase abspaltet.

5. Verfahren zur Herstellung von Proteinen mit N-terminalen Prolin aus Fusionsproteinen, die die N-terminale Tetrapeptidsequenz Pro-Xyz-Gly-Pro mit Xyz in der obenangegebenen Bedeutung enthalten, dadurch gekennzeichnet, dass man die Xyz-Gly-Bindung in der Tetrapeptidsequenz mit einer Collagenase selektiv spaltet und anschliessend den Glycin-Rest mit einer Aminoacylprolin-aminopeptidase entfernt.

6. Verfahren zur Herstellung von Fremdproteinen, deren zweite N-terminale Aminosäure Prolin ist, dadurch gekennzeichnet, dass man eine weitere Aminosäure Zxy, die jede beliebige Aminosäure ausser Prolin darstellen kann, zwischen der N-terminalen Tetrapeptidsequenz Pro-Xyz-Gly-Pro des Fusionsproteins, wobei Xyz jede beliebige Aminosäure darstellen kann, und dem gewünschten Fremdprotein einfügt und nach Collagenase-Spaltung der Xyz-Gly-Bindung die Postprolindipeptidylaminopeptidase-Spaltung der Pro-Zxy-Bindung durchführt, an die sich anschliessend die Abtrennung der Aminosäure Zxy mit Leucinaminopeptidase anschliesst.

7. Verfahren zur Herstellung von Proteinen, deren erste N-terminale Aminosäure nicht Prolin ist oder deren erste und zweite N-terminale Aminosäuren nicht Aminoacyl-Prolin sind, dadurch gekennzeichnet, dass man von einem Fusionsprotein, das die N-terminale Dipeptidsequenz Met-Pro enthält, den Met-Pro-Rest mit einer Postprolindipeptidylaminopeptidase abspaltet.

8. Verfahren zur Herstellung von Proteinen, dadurch gekennzeichnet, dass man von einem Fusionsprotein, das N-terminal die Tripeptidsequenz Yzx-Pro-Vwu enthält, wobei Yzx jede beliebige Aminosäure und Vwu bis auf Prolin jede beliebige Aminosäure und bis auf Aminoacylprolin jedes beliebige Dipeptid bedeuten können, die C-terminal auf die Dipeptidsequenz Yzx-Pro folgende Vwu-Protein-Sequenz enzymatisch abspaltet.

9. Verfahren zur Herstellung von Proteinen nach Anspruch 8, dadurch gekennzeichnet, dass man die Pro-Vwu-Bindung in der Tripeptidsequenz Yzx-Pro-Vwu mit einer Postprolindipeptidylaminopeptidase spaltet.

## Claims

1. Process for the manufacture of proteins, characterised in that the protein sequence C-terminally following the tetrapeptide sequence is enzymatically split off from a fusion protein which contains the N-terminal tetrapeptide sequence Pro-Xyz-Gly-Pro in which Xyz can represent any desired amino acid. .

2. Process for the manufacture of proteins according to claim 1, characterised in that the Xyz-Gly-bond in the tetrapeptide sequence is selectively split using a collagenase and subsequently the glycine radical is removed using an aminoacylproline aminopeptidase and the proline radical is removed using a proline aminopeptidase.

3. Process for the manufacture of proteins according to claim 2, characterised in that the splitting off of the glycine radical using an aminoacylproline aminopeptidase and the splitting off of the proline radical using proline aminopeptidase is carried out in one step.

4. Process for the manufacture of proteins according to claim 2, characterised in that the Gly-Pro radical is split off using a post-proline dipeptidylaminopeptidase.

5. Process for the manufacture of proteins having N-terminal proline from fusion proteins which contain the N-terminal tetrapeptide sequence Pro-Xyz-Gly-Pro in which Xyz has the meaning given above, characterised in that the Xyz-Gly-bond in the tetrapeptide sequence is selectively split using a collagenase and subsequently the glycine radical is removed using an aminoacylproline aminopeptidase.

6. Process for the manufacture of foreign proteins the second N-terminal amino acid of which is proline, characterised in that a further amino acid Zxy which can represent any desired amino acid with the exception of proline is introduced between the N-terminal tetrapeptide sequence Pro-Xyz-Gly-Pro of the fusion protein, it being possible for Xyz to represent any desired amino acid, and the desired foreign protein and, after collagenase splitting of the Xyz-Gly-bond, the post-proline dipeptidylaminopeptidase splitting of the Pro-Xyz-bond is carried out, followed subsequently by the splitting off of the amino acid Zxy using leucine aminopeptidase.

7. Process for the manufacture of proteins the first N-terminal amino acid of which is not proline or the first and second N-terminal amino acids of which are not aminoacyl proline, characterised in that the Met-Pro-radical is split off from a fusion protein which contains the N-terminal dipeptide sequence Met-Pro using a postproline dipeptidylaminopeptidase.

8. Process for the manufacture of proteins, characterised in that the Vwu-protein sequence C-terminally following the dipeptide sequence Yzx-Pro is split off enzymatically from a fusion protein which contains in the N-terminal position the tripeptide sequence Yzx-Pro-Vwu in which Yzx can represent any desired amino acid and Vwu can represent any desired amino acid with the exception of proline and any desired dipeptide with the exception of aminoacylproline.

9. Process for the manufacture of proteins according to claim 8, characterised in that the Pro-Vwu-bond in the tripeptide sequence Yzx-Pro-Vwu is split using a post-proline dipeptidylaminopeptidase.

**Revendications**

1. Procédé de préparation de protéines, procédé caractérisé en ce que, partant d'une protéine de fusion contenant, à son extrémité amino, une séquence tétrapeptidique Pro-Xyz-Gly-Pro dans laquelle Xyz peut désigner n'importe quel aminoacide, on en sépare par voie enzymatique la séquence protéinique qui fait suite à la séquence tétrapeptidique du côté de son extrémité carboxy.

2. Procédé de préparation de protéines selon la revendication 1, procédé caractérisé en ce qu'on coupe sélectivement, au moyen d'une collagénase, la liaison Xyz-Gly contenue dans la séquence tétrapeptidique, puis on élimine le radical de glycine au moyen d'une aminoacylproline-aminopeptidase et le radical de proline au moyen d'une proline-aminopeptidase.

3. Procédé de préparation de protéines selon la revendication 2, caractérisé en ce que l'élimination du radical de glycine au moyen d'une aminoacylproline-aminopeptidase et l'élimination du radical de proline au moyen d'une proline-aminopeptidase sont effectuées en une seule étape.

4. Procédé de préparation de protéines selon la revendication 2, caractérisé en ce qu'on élimine le radical Gly-Pro au moyen d'une postproline-dipeptidylaminopeptidase.

5. Procédé de préparation de protéines renfermant la proline à leur extrémité amino (proline «N-terminale») à partir de protéines de fusion contenant la séquence tétrapeptidique Pro-Xyz-Gly-Pro N-terminale (dans laquelle Xyz a la signification précédemment donnée), procédé caractérisé en ce qu'on coupe sélectivement, au moyen d'une collagénase, la liaison Xyz-Gly contenue dans la séquence tétrapeptidique, puis on élimine le radical de glycine au moyen d'une aminoacylproline-aminopeptidase.

6. Procédé de préparation de protéines étrangères dont le deuxième aminoacide à partir de l'extrémité amino est la proline, procédé caractérisé en ce qu'on insère un aminoacide supplémentaire Zxy, qui peut être n'importe quel aminoacide sauf la proline, entre la séquence tétrapeptidique Pro-Xyz-Gly-Pro N-terminale de la protéine de fusion, dans laquelle Xyz peut représenter n'importe quel aminoacide, et la protéine étrangère voulue, et, après avoir coupé la liaison Xyz-Gly au moyen d'une collagénase, on coupe la liaison Pro-Zxy au moyen d'une postprolinedipeptidylaminopeptidase, après quoi on élimine l'aminoacide Zxy au moyen d'une leucine-aminopeptidase.

7. Procédé de préparation de protéines dont le premier aminoacide à compter de l'extrémité amino n'est pas la proline ou dont les premier et deuxième aminoacides, à compter de l'extrémité amino, ne sont pas des aminoacyl-prolines, procédé caractérisé en ce que, partant d'une protéine de fusion qui contient la séquence dipeptidique Met-Pro à son extrémité amino, on en élimine le radical Met-Pro au moyen d'une postproline-dipeptidylaminopeptidase.

8. Procédé de préparation de protéines, procédé caractérisé en ce que, partant d'une protéine de fusion contenant la séquence tripeptidique Yzx-Pro-Vwu N-terminale (dans laquelle Yzx peut représenter n'importe quel aminoacide, et Vwu peut représenter n'importe quel aminoacide sauf la proline ou n'importe quel dipeptide sauf une aminoacylproline), on en élimine par voie enzymatique la séquence Vwu-protéine qui fait suite à la séquence dipeptidique Yzx-Pro du côté de son extrémité carboxy.

9. Procédé de préparation de protéines selon la revendication 8, caractérisé en ce qu'on coupe la liaison Pro-Vwu contenue dans la séquence tripeptidique Yzx-Pro-Vwu au moyen d'une post-proline-dipeptidylaminopeptidase.